(19) Europäisches Patentamt
European Patent Office
Office européen des brevets

(11) **EP 1 923 094 B1**

(12) **EUROPÄISCHE PATENTSCHRIFT**

(45) Veröffentlichungstag und Bekanntmachung des Hinweises auf die Patenterteilung:
**28.12.2011 Patentblatt 2011/52**

(51) Int Cl.:
***A61N 1/16*** *(2006.01)*

(21) Anmeldenummer: **07020556.2**

(22) Anmeldetag: **20.10.2007**

(54) **Elektrodenkatheter zu Interventionszwecken**

Electrode catheter for intervention purposes

Cathéter à électrode destiné à des interventions

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **17.11.2006 DE 102006054620**
**02.07.2007 DE 102007030678**
**26.07.2007 DE 102007034990**

(43) Veröffentlichungstag der Anmeldung:
**21.05.2008 Patentblatt 2008/21**

(73) Patentinhaber: **Biotronik CRM Patent AG**
**6341 Baar (CH)**

(72) Erfinder: **Geistert, Wolfgang, Dr.**
**79618 Rheinfelden (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
**WO-A-2007/118194      US-A1- 2002 138 102**
**US-A1- 2002 198 569      US-A1- 2006 009 819**

EP 1 923 094 B1

**Beschreibung**

Die vorliegende Erfindung betrifft einen Elektrodenkatheter zu Interventionszwecken, wie Herzschrittmacher-, Neurostimulations-, ICD-Elektroden- oder EP-Katheter, gemäß dem Oberbegriff des Anspruches 1. Unter dem Begriff "Elektrodenkatheter" sollen dabei alle Arten von langgestreckten Implantaten verstanden werden, die mindestens einen im langgestreckten Körper des Implantats eingeschlossenen und dort mindestens einen vom den Patienten umgebenden Medium weitgehend isoliert verlaufenden Draht umfassen, welcher in der Nähe des distalen Endes in direktem oder indirektem elektrischen Kontakt zum umgebenden Medium (z.B. Körpergewebe) steht. Neben den obengenannten Arten können z.B. noch elektrophysiologische Diagnostikkatheter, Ablationskatheter und Schrittmacherelektroden genannt werden.

Derartige Elektrodenkatheter weisen bekanntermaßen einen lang gestreckten Körper mit einem distalen und einem proximalen Ende auf. Im Bereich des distalen Endes ist mindestens ein Elektrodenpol vorgesehen, welcher typischerweise der Abgabe von Interventionspulsen oder der Wahrnehmung von Herz-, Nerven- oder Hirnsignalen dient. Dieser Elektrodenpol ist beispielsweise als direkt am distalen Ende angeordneter Tip-Elektrodenpol, als mit Abstand davon platzierter Ringpol oder als Schockelektrode ausgelegt. Die darüber abgegebenen Interventionspulse sind beispielsweise die Schrittmacherpulse eines Herzschrittmachers bzw. Neurostimulators, ein Hochspannungspuls im Falle eines Defibrillators oder ein Ablationsenergiepuls im Falle eines Ablationsgerätes.

Im Elektrodenkörper verläuft eine isolierte Zuleitung zu diesem Elektrodenpol. Ferner weisen solche Elektrodenkatheter meist weitere, allgemein so zu bezeichnende Elektrodenpole auf, über welche die Zuleitung mit Gewebe in elektrischen Kontakt treten kann. Als Beispiele sind ein Ring-Elektrodenpol einer bipolaren Elektrode oder eines EP-Katheters zu nennen. Ferner ist eine die mindestens eine Zuleitung umgebende Elektrodenummantelung im Elektrodenkörper zur Isolation der Zuleitung vorgesehen.

In den letzten Jahren haben nun Magnetresonanz-Diagnosegeräte wegen ihrer patientenschonenden, nicht-invasiven und völlig schmerz- sowie nebenwirkungsfreien Untersuchungsmethodik erheblich an Bedeutung gewonnen. Übliche Elektrodenkatheter zeigen dabei die Problematik, dass derartige Elektrodenkatheter das Gewebe in Magnetresonanz-Diagnosegeräten unter dem Einfluss der davon generierten elektromagnetischen Strahlung aufgrund elektromagnetischer Induktion und der Abgabe der induzierten Energie im Bereich ihrer Kontaktfläche(n) zum Gewebe stark erwärmen. Der Grund hierfür liegt insbesondere in den massiven, metallischen Zuleitungen zu den Elektrodenpolen, die als Antenne wirken und bei denen aufgrund ihrer Isolierung die durch Hochfrequenz-(HF)-Felder induzierten Antennenströme nur an den Elektrodenpolen, die die elektrische Grenzfläche zum Gewebe bilden, in den Körperelektrolyten abgeleitet werden. Die erwähnten HF-Felder arbeiten beispielsweise in einem Arbeitsfrequenzbereich von 64 MHz bei einem 1,5-Tesla-MR-Tomographen. Da eine extrem starke Erhitzung des Gewebes in der Nähe der Elektrodenpole auftreten kann, ist Trägern von kardiologischen und neurologischen Interventionsgeräten, wie Herzschrittmachern, Neurostimulatoren oder Defibrillatoren, der Zugang zu Magnetresonanz-Diagnosegeräten in aller Regel versperrt. Ebenso wenig sind elektrophysiologische Untersuchungen und Interventionen, wie z.B. Ablationen, in MR-Geräten möglich.

Um die gefährliche Erwärmung der Körperzellen zu verhindern bzw. zu minimieren, muss der maximale Antennenstrom limitiert bzw. reduziert werden. Bekannte Lösungen schlagen hierzu diskrete Bauelemente vor, welche als Bandsperre oder als Tiefpassfilter wirken und so für die interessierenden Frequenzen den Längsstrom der Antenne limitieren anders ausgedrückt den Längswiderstand der Antenne erhöhen. Andere Lösungen schlagen Kondensatoren vor, die parallel zur Isolation geschaltet sind und so den Antennenstrom ableiten.

Dokument WO-A-2007/118194 offenbart einen Stand der Technik im Sinne des Artikels 54(3) EPÜ (siehe insbesondere Figuren 88, 89).

Hierzu seien beispielsweise die US 6,944,489, die US 2003/0144720, die US 2003/0144721, die US 2005/0288751 A1 (und die im Wesentlichen gleichlautenden, gleichzeitig veröffentlichten Parallel-Schriften US 2005/0288752 A1, US 2005/0288754 A1 und US 2005/0288756 A1) angeführt.

Die US 2006/0009819 A1 offenbart einen Herzschrittmacher mit einer lang gestreckten Elektrode, die mit einem Pulsgenerator-Konnektor verbunden ist. Dabei ist ein passiver Verlustschaltkreis vorgesehen, der elektrisch zwischen einem distalen Abschnitt der Elektrodenzuleitung und einer demgegenüber Hochfrequenz-geerdeten Oberfläche geschaltet ist. Der passive Verlustschaltkreis weist eine Hochfrequenz-Impedanz auf, die etwa gleich einer charakteristischen Impedanz der Elektrode in ihrem Implantationszustand im Körper ist. Dadurch wird an den Klemmen des Verlustschaltkreises die Reflexion einfallender Wellen minimiert und deren Energie gezielt hier dissipiert. Der passive Verlustschaltkreis wirkt weiterhin als Tiefpass-Filter, wodurch die Elektrode im Normalbetrieb des Herzschrittmachers funktionsfähig ist. Dieses Dokument offenbart den nächstliegenden Stand der Technik im Sinne des Artikels 54(2) EPÜ.

Nachteil der bekannten Lösungen ist, dass diskrete Bauelemente die Herstellung verkomplizieren und damit teuer machen. Außerdem reduzieren solche diskreten Bauelemente die Zuverlässigkeit und Langzeitstabilität von Elektrodenkathetern, was insbesondere dann nachteilig ist, wenn diese als Langzeitimplantate vorgesehen sind. Und schließlich erfordern diskrete Bauelemente, wie Induktivitäten und Widerstände, eine gewisse Baugröße, wenn sie für Hochstromanwendungen, wie Defibrillation und HF-Ablation vorgesehen sind. Dies steht im Widerspruch zu den Bestrebungen, ein Implantat besonders klein und schlank zu gestalten.

Ausgehend von der geschilderten Problematik liegt der Erfindung die Aufgabe zugrunde, Elektrodenkatheter so auszubilden, dass sie konstruktiv einfach gestaltet und preiswert herstellbar sowie in Strahlungsfeldern von Magnetresonanz-Diagnosegeräten ohne relevantes Risiko für den Träger platzierbar sind. Insbesondere sollen diskrete Bauelemente zur Lösung der Problematik vermieden und die Eigenschaften der Elektrode hinsichtlich ihrer Antennencharakteristik auf andere Art so beeinflusst werden, dass es nicht zu Stromkonzentrationen und entsprechend nicht zu übermäßigen Erwärmungen an Elektrodenpolen und insbesondere nicht um die Spitze des Elektrodenkatheters herum kommen kann. Der geschaffene Elektrodenkatheter soll so auch für Hochstromanwendungen (ICD, Ablation) geeignet sein.

Diese Aufgabe wird durch eine Vorrichtung gemäß Anspruch 1 gelöst.

Durch diese Auslegung findet im Hinblick auf die gestellte Aufgabe eine Insensitivierung des Elektrodenkatheters gegen die Einstrahlung eines elektromagnetischen Feldes einer Störfrequenz mit einer bestimmten Wellenlänge statt, ohne dass der Elektrodenkatheter gesonderter diskreter Bauelemente bedürfte.

Der physikalische Hintergrund der Erfindung stellt sich wie folgt dar:

Ein langgestreckter, elektrischer Leiter wird bei einer bestimmten Frequenz f zu einer schlechten Antenne, wenn er eine Länge aufweist, die kleiner ist als die Hälfte, besser jedoch ein Viertel der Wellenlänge $\lambda$ bei dieser Frequenz. Die Wellenlänge hängt dabei gemäß folgender Formel von dem umgebenden Material - insbesondere von dessen Dielektrizität (Permittivität) e und Permeabilität $\mu$- ab, in dem sich der Leiter befindet:

$$\lambda = \frac{1}{f \times \sqrt{\mu\varepsilon}}$$

Die Dielektrizität (Permittivität) $\varepsilon$ setzt sich dabei aus der Naturkonstante $\varepsilon_0$ (8,854...·10$^{-12}$ As/Vm) und der relativen Dielektrizität (Permittivität) $\varepsilon_R$ zusammen ($\varepsilon = \varepsilon_0 \times \varepsilon_R$). Die Permeabilität $\mu$ ergibt sich aus der Naturkonstante $\mu_0$ (1,2566...· 10$^{-6}$ kg·m/A$^2$s$^2$) und der relativen Permeabilität $\mu_R$ ($\mu = \mu_0 \times \mu_R$).

Für Körpergewebe, welches hauptsächlich aus Wasser ($\mu_R$ ca. 80) besteht, kommt man so bei einer für 1,5 Tesla-MR-Geräte typischen Frequenz von 64 MHz auf eine Wellenlänge $\lambda$ von ca. 52 cm. $\lambda/4$ beträgt also etwa 13 cm.

In einem erfindungsgemäßen Elektrodenkatheter wird also die Zuleitung in kleine Abschnitte eingeteilt, die jeweils kürzer als die halbe, vorzugsweise als ein Viertel der Wellenlänge der Störstrahlung ist, gegen die der Elektrodenkatheter insensitiv gemacht werden soll.

Je nach Grad der geforderten Insensitivität können sich diese Richtungswechsel über die gesamte Länge des Elektrodenkatheters oder auch nur über eine Teillänge erstrecken. Die größte Insensitivität wird erreicht, wenn sich die Richtungswechsel über die gesamte Länge des Elektrodenkatheters erstrecken.

Nun ist es nicht so, dass in kurzen, also kleiner als $\lambda/4$ langen Antennen, die sich in einem elektromagnetischen Feld befinden, gar keine Spannung induziert wird. Deshalb ist es von Vorteil, wenn die Länge der einzelnen Schenkel so bemessen wird, dass sich die in den einzelnen Schenkeln induzierten Ströme durch ihre Phasenverschiebung gerade aufheben. Dazu müssen diese doch eine gewisse Länge aufweisen. Die Strecke zwischen zwei Richtungswechseln soll daher zwar einerseits kürzer als $\lambda/2$, aber andererseits nicht kürzer als $\lambda/16$ der Wellenlänge der Störfrequenz sein, bei der der Elektrodenkatheter insensitiv sein soll.

Als vorteilhaft hat sich eine Konstruktion erwiesen, bei der die langen Schenkel der durch die Richtungswechsel geschaffenen Abschnitte der Zuleitung eine Länge aufweisen, die etwa $\lambda/4$ entspricht und bei der die kurzen Schenkel eine Länge aufweisen, die etwa $\lambda/8$ entspricht. Werden die Schenkel kürzer als diese Werte, so heben sich die induzierten Spannungen zunehmend weniger ideal auf, die Dämpfungswirkung der Anordnung verschlechtert sich und die an den Enden des Elektrodenkatheters austretenden Ströme werden wieder größer als bei der genannten Idealkonstruktion.

Gemäß weiteren bevorzugten Ausführungsformen kann die Zuleitung zusätzlich verdrillt oder gewendelt gelegt sein. Die Zuleitung selbst ist grundsätzlich durch jedweden elektrischen Leiter realisierbar, also beispielsweise als Draht, Litze, Wendel oder gedruckte Schaltung auf einem entsprechend konfigurierten Substrat.

Der erfindungsgemäße Elektrodenkatheter ist schließlich im Zusammenhang mit seiner Zuleitungsisolation im Hinblick auf eine weitergehende Unempfindlichkeit gegenüber Hochfrequenz-Störfeldern und verbesserte Einsatzfähigkeit in Strahlungsfeldern von Magnetresonanz-Diagnosegeräten optimierbar, indem die Isolation so gestaltet wird, dass sie bei der Störfrequenz eine gute Kopplung der Zuleitung zum umgebenden Körpergewebe gewährleistet. Dazu kann die Isolation sehr dünn sein, was eine kapazitive Kopplung erlaubt, oft aber aus Gründen der mechanischen Widerstandsfähigkeit ausgeschlossen ist. Alternativ oder zusätzlich kann die Isolation der Zuleitung auch aus einem minderisolierenden oder frequenzabhängig isolierendem Material hergestellt werden. Diese Maßnahme ist Gegenstand der älteren Anmeldung DE 10 2007 022 333.3 der Anmelderin. Kurz zusammengefasst wird durch diese Maßnahme die Güte des aus dem Elektrodenkatheter mit dem Körper gebildeten Schwingkreises soweit reduziert, dass einerseits die von der Antenne aufgenommene Energie vermindert wird und dass sich andererseits die Verluste in dem Gesamtgebilde Elektrodenzuleitung/Isolation/Körper so verteilen, dass es zu keiner übermäßigen Stromkonzentration an bestimmten Punkten kommt.

Weitere Merkmale, Einzelheiten und Vorteile der Erfindung ergeben sich aus der nachfolgenden Beschreibung, in der Ausführungsbeispiele anhand der beigefügten Zeichnungen näher erläutert werden. Es zeigen:

Fig. 1 und 2    schematische Längsschnitte von Elektrodenkathetern in zwei unterschiedlichen Ausführungsformen,

Fig. 3    Detailansichten von Zuleitungen an den Stellen ihrer Richtungswechsel in unterschiedlichen Konfigurationen, und

Fig. 4    einen schematischen Längsschnitt eines Elektrodenkatheters nach dem Stand der Technik.

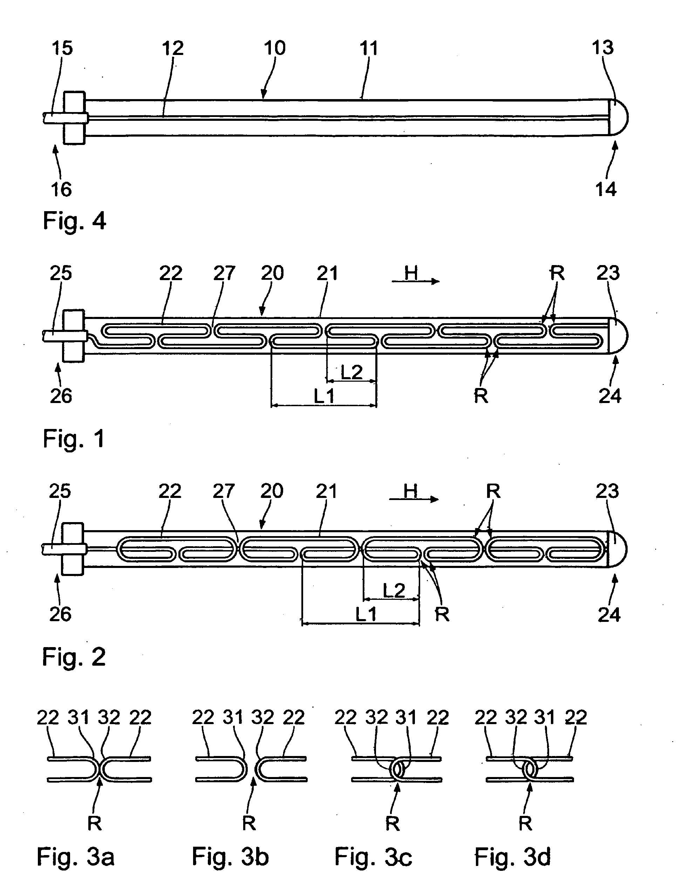

Fig. 4 zeigt schematisch den Stand der Technik in Form eines Längsschnitts durch einen Elektrodenkatheter 10. Die Zuleitung 12 ist im Wesentlichen gestreckt im Schaft 11 des Elektrodenkatheters 10 untergebracht. Die Zuleitung verbindet den Elektrodenpol 13 am distalen Ende 14 des Elektrodenkatheters mit dem die elektrische Verbindung zu einem Gerät herstellenden Konnektor 15 am proximalen Ende 16 des Elektrodenkatheters 10. Als Gerät werden Therapiegeräte wie beispielsweise implantierbare oder externe Stimulatoren, Herzschrittmacher oder Defibrillatoren angesehen. Denkbar sind aber auch Diagnosegeräte oder Kombinationen aus Therapie- und Diagnosegeräten

Die Fig. 1 gibt schematisch eine Ausführungsformen der Anordnung einer Zuleitung 22 im Schaft 21 eines erfindungsgemäßen Elektrodenkatheters 20 wieder. Der in Figur 2 gezeigte Elektrodenkatheter ist nicht Teil der Erfindung. Die Zuleitung verbindet den Elektrodenpol 23 am distalen Ende 24 des Elektrodenkatheters mit dem Konnektor 25 am proximalen Ende 26 des Elektrodenkatheters. In Fig. 1 ist eine mäanderförmige Anordnung dargestellt, in der die Zuleitung 22 mehrfach ihre Verlaufsrichtung in und gegen der Hauptrichtung H des Elektrodenkatheters 20 wechselt. Die damit gebildeten Richtungswechsel R führen folglich zu langen Schenkeln L1 und kurzen Schenkeln L2 in der Zuleitung 22. Die Länge des langen Schenkels L1 entspricht etwa einem Viertel der Wellenlänge der Störfrequenz, also $\lambda/4$. Die Länge des kurzen Schenkels L2 entspricht etwa $\lambda/8$ der Wellenlänge der Störfrequenz.

Für einen Elektrodenkatheter, der für eine Frequenz von 64 MHz insensitiv sein soll, verläuft also die isolierte Zuleitung 22 beispielsweise beginnend von der distalen Spitze 7 cm in proximale Richtung, dann wird die Zuleitung 22 ca. 180° gefaltet und verläuft wieder 6,5 cm in distale Richtung, dann wird die Zuleitung 22 wieder gefaltet und verläuft jetzt 13 cm in proximale Richtung, dann wieder 6,5 cm zurück in distale Richtung, dann wieder 13 cm in proximale Richtung usw., bis die Zuleitung 22 das proximale Ende 25 des Elektrodenkatheters 20 erreicht.

In Fig. 2 ist eine schlaufenförmige Konfiguration der Zuleitung 22 gezeigt, bei der die einzelnen Schenkel L1 und L2 der Zuleitung 22 durch entsprechende Richtungswechsel R die erkennbare Schlaufenform erhalten. Im Übrigen gelten für die Antennenwirkung der Zuleitung die Ausführungen zu Fig. 1.

Für beide Varianten gemäß Fig.1 und 2 gilt ferner, dass die als Ganzes mit 27 bezifferte Isolation der Zuleitung 22 aus einem frequenzabhängig isolierenden Material besteht, wodurch sich die Güte der Antennenwirkung verschlechtert und der Elektrodenkatheter weniger Störenergie aufnimmt und sich außerdem eine weitere Dissipation und damit Verteilung der in den Elektrodenkatheter durch eine Störfrequenz eingestrahlten Energie einstellt.

In weiteren, nicht dargestellten Ausführungsformen sind die Schenkel der Zuleitungsmäander bzw. -schlaufen miteinander verdrillt oder die gesamte, wie eben beschrieben gelegte Zuleitung ist zu einer Wendel geformt.

Es ist ferner dafür zu sorgen, dass sich die nebeneinander liegenden und sich kreuzenden Schenkel und Spitzen der Zuleitung 22 nicht galvanisch berühren, um einen Kurzschluss zu verhindern. Dies kann dadurch geschehen, dass die Zuleitungen eine individuelle Isolation z.B. aus Lack oder aus einem Polymer (z.B. eine Teflonbeschichtung) erhalten. In einer anderen Ausführungsform verlaufen die Schenkel in verschiedenen Lumen des Katheterschaftes. Die individuelle Isolation der Zuleitung 22 ist in den Figuren nicht dargestellt.

Die Zuleitung 22 selbst kann zum Beispiel ein Draht, eine Litze aus mehreren Drähten, eine Wendel aus einem oder mehreren Drähten oder eine auf einem Träger aufgebrachte, gedruckte Verdrahtung sein. Die Mäander bzw. Schlaufen können durch entsprechende Führung des Drahtes bzw. der Litze oder durch Verbinden der Enden einzelner, gestreckter oder gewendelter Leiterstücke der Schenkellänge L1 bzw. L2 mittels Löten, Schweißen, Crimpen etc. hergestellt werden.

Fig. 3 zeigt schematisch mögliche Ausführungsformen der Mäander/Schlaufen-Enden-Konfiguration eines Elektrodenkatheters 20 im Bereich zweier benachbarter Richtungswechsel R der Zuleitung 22. In Fig. 3a berühren sich die Spitzen 31, 32 der Mäander/Schlaufen-Enden. In Fig. 3b berühren sich die Spitzen 31, 32 der Mäander/Schlaufen-Enden nicht. In Fig. 3c überlappen sich die Spitzen 31, 32 der Mäander/Schlaufen-Enden, während in Fig. 3d die Spitzen 31, 32 der Mäander/Schlaufen-Enden ineinander verschlungen sind. In allen Fällen ist jedoch auf die oben erörterte galvanische Trennung zwischen den Windungen der Zuleitung 22 zu achten.

**Patentansprüche**

1. Elektrodenkatheter zu Interventionszwecken, wie Herzschrittmacher-, Neurostimulations-, ICD-Elektroden- oder EP-Katheter, umfassend

   - einen langgestreckten Elektrodenkatheterkörper (21) mit einem distalen Ende (24) und einem proximalen Ende (26),
   - mindestens einen Elektrodenpol (23) im Bereich des distalen Endes (24) des Elektrodenkörpers (20), und
   - mindestens eine im Elektrodenkörper (21) in einer Hauptrichtung (H) von proximal nach distal isoliert verlaufende Zuleitung (22) zu dem mindestens einen Elektrodenpol (23),

   wobei

   - zur Insensitivierung des Elektrodenkatheters gegen die Einstrahlung eines elektromagnetischen Feldes einer Störfrequenz mit einer bestimmten Wellenlänge ($\lambda$) die Zuleitung (22) mindestens zweimal derart ihre Verlaufsrichtung wechselt, dass sie mindestens einmal entgegen der Hauptrichtung (H) verläuft und dass der Abstand (L1, L2) zwischen zwei Richtungswechseln (R) kürzer als die halbe Wellenlänge ($\lambda$/2) der Störfrequenz ist, die Zuleitung (22) vielfache Richtungswechsel (R) entlang ihres Verlaufes aufweist, und die Zuleitung (22) durch die vielfache Richtungswechsel (R) in abewechselnd kurze und lange Schenkel (L1, L2) eingeteilt ist, und die Zuleitung in ihrem Verlauf aufgrund der Richtungswechsel mäanderförmig gelegt ist.

2. Elektrodenkatheter nach Anspruch 1, **dadurch gekennzeichnet, dass** der Abstand (L1, L2) zwischen zwei Richtungswechseln (R) maximal einem Viertel ($\lambda$/4) der Wellenlänge ($\lambda$) der Störfrequenz entspricht.

3. Elektrodenkatheter nach einem der vorgenannten Ansprüche, **dadurch gekennzeichnet, dass** der Abstand (L1, L2) zwischen zwei aufeinanderfolgenden Richtungswechseln (R) der Zuleitung (22) größer als ein Sechzehntel ($\lambda$/16) der Wellenlänge ($\lambda$) der Störfrequenz ist. die Zuleitung (22) durch die vielfachen Richtungswechsel (R) in abwechselnd kurze und lange Schenkel (L1, L2) eingeteilt ist,

4. Elektrodenkatheter nach Anspruch 1, **dadurch gekennzeichnet, dass** die Zuleitung (22) in ihrem Verlauf zusätzlich verdrillt oder gewendelt ist.

5. Elektrodenkatheter nach einem der vorgenannten Ansprüche, **dadurch gekennzeichnet, dass** die Zuleitung (22) als Draht, Litze, Wendel oder gedruckte Schaltung ausgeführt ist.

6. Elektrodenkatheter nach einem der vorgenannten Ansprüche, **dadurch gekennzeichnet, dass** im Bereich zweier benachbarter Richtungswechsel (R) die Windungen der Zuleitungen (22) sich nicht berühren, berühren, überlappen oder ineinander verschlungen sind.

7. Elektrodenkatheter nach einem der vorgenannten Ansprüche, **dadurch gekennzeichnet, dass** die Isolation (27) der Zuleitung (22) aus einem minder- oder frequenzabhängig isolierenden Material besteht.

**Claims**

1. An electrode catheter for intervention purposes, such as a cardiac pacemaker catheter, a neurostimulation catheter, an ICD electrode catheter or an EP catheter, comprising

   - an elongated electrode catheter body (21) having a distal end (24) and a proximal end (26),
   - at least one electrode pole (23) in the region of the distal end (24) of the electrode body (20), and
   - at least one feed line (22) to the at least one electrode pole (23), the feed line running in the electrode body (21) in a primary direction (H) from proximal to distal in an insulated manner,

   wherein

   - the feed line (22), so as to desensitize the electrode catheter with respect to the irradiation of an electromagnetic field of an interference frequency having a defined wavelength ($\lambda$), changes the direction of the course thereof at least twice such that it runs at least once counter to the primary direction (H) and the distance (L1, L2) between two directional changes (R) is shorter than half the wavelength ($\lambda$/2) of the interference frequency, the feed line (22) has multiple directional changes (R) along the course thereof, and the feed line (22) is divided into alternating short and long limbs (L1, L2) by the multiple directional changes (R), and the course of the feed line has a meander shape as a result of the directional changes.

2. The electrode catheter according to claim 1, **char-**

**acterized in that** the distance (L1, L2) between two directional changes (R) is no more than one quarter (λ/4) of the wavelength (λ) of the interference frequency.

3. An electrode catheter according to either one of the preceding claims, **characterized in that** the distance (L1, L2) between two consecutive directional changes (R) of the feed line (22) is greater than one sixteenth (λ/16) of the wavelength (λ) of the interference frequency.

4. The electrode catheter according to claim 1, **characterized in that** the feed line (22) is additionally twisted or coiled over the course thereof.

5. An electrode catheter according to any one of the preceding claims, **characterized in that** the feed line (22) is designed as a wire, strand, coil or printed circuit board.

6. An electrode catheter according to any one of the preceding claims, **characterized in that** the windings of the feed lines (22) in the region of two adjoining directional changes (R) are not in contact with each other, are in contact with each other, overlap each other or are intertwined with each other.

7. An electrode catheter according to any one of the preceding claims, **characterized in that** the insulation (27) of the feed line (22) comprises a lesser insulating material or a material that provides insulation depending on the frequency.

**Revendications**

1. Cathéter à électrode destiné à des interventions, tel qu'un cathéter de stimulateur cardiaque, un cathéter de neurostimulation, un cathéter à électrode de défibrillateur automatique implantable (DAI) ou un cathéter d'électrophysiologie (EP), comprenant

   - un corps de cathéter à électrode allongé (21), avec une extrémité distale (24) et une extrémité proximale (26),
   - au moins un pôle d'électrode (23) dans la région de l'extrémité distale (24) du corps d'électrode (20), et
   - au moins une ligne d'alimentation (22) isolée parcourant le corps d'électrode (21) dans une direction principale (H) allant de l'extrémité proximale à l'extrémité distale, vers l'au moins un pôle d'électrode (23),

   dans lequel

   - pour l'insensibilisation du cathéter à électrode contre le rayonnement d'un champ électromagnétique d'une fréquence parasite avec une certaine longueur d'onde (λ), la ligne d'alimentation (22) modifie au moins deux fois sa direction de parcours, de sorte qu'elle s'étend au moins une fois inversement à la direction principale (H) et que l'écart (L1, L2) entre deux changements de direction (R) est plus court que la demi-longueur d'onde (λ/2) de la fréquence parasite,
   - la ligne d'alimentation (22) comporte des changements de direction multiples (R) le long de son parcours, et
   - la ligne d'alimentation (22) est divisée en branches alternativement courtes et longues (L1, L2) par les changements de direction multiples (R), et la ligne d'alimentation est disposée en forme de méandres dans son parcours, en raison des changements de direction.

2. Cathéter à électrode selon la revendication 1, **caractérisé en ce que** l'écart (L1, L2) entre deux changements de direction (R) correspond au maximum à un quart (λ/4) de la longueur d'onde (λ) de la fréquence parasite.

3. Cathéter à électrode selon l'une des revendications précédentes, **caractérisé en ce que** l'écart (L1, L2) entre deux changements de direction consécutifs (R) de la ligne d'alimentation (22) est supérieur à un seizième λ/16) de la longueur d'onde (λ) de la fréquence parasite.

4. Cathéter à électrode selon la revendication 1, **caractérisé en ce que** la ligne d'alimentation (22) est en outre torsadée ou hélicoïdale dans son parcours.

5. Cathéter à électrode selon l'une des revendications précédentes, **caractérisé en ce que** la ligne d'alimentation (22) est conçue comme un fil, un fil toronné, un filament ou un circuit imprimé.

6. Cathéter à électrode selon l'une des revendications précédentes, **caractérisé en ce que** dans la région de deux changements de direction consécutifs (R), les méandres de la ligne d'alimentation (22) ne se touchent pas, ne se chevauchent pas et ne s'entrelacent pas.

7. Cathéter à électrode selon l'une des revendications précédentes, **caractérisé en ce que** l'isolation (27) de la ligne d'alimentation (22) est constituée d'un matériau moins isolant où isolant fonction de la fréquence.

Fig. 4

Fig. 1

Fig. 2

Fig. 3a          Fig. 3b          Fig. 3c          Fig. 3d

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007118194 A
- US 6944489 B
- US 20030144720 A
- US 20030144721 A
- US 20050288751 A1
- US 20050288752 A1
- US 20050288754 A1
- US 20050288756 A1
- US 20060009819 A1
- DE 102007022333